(19) **Europäisches Patentamt**

**European Patent Office**

**Office européen des brevets**

(11) **EP 0 608 593 B1**

(12) **EUROPEAN PATENT SPECIFICATION**

(45) Date of publication and mention
of the grant of the patent:
**28.06.2006 Bulletin 2006/26**

(51) Int Cl.:
*A61B 5/20* (2006.01)    *A61B 5/22* (2006.01)

(21) Application number: **93300584.5**

(22) Date of filing: **27.01.1993**

(54) **Method and system for on-line measurement, storage, retrieval and analysis of urodynamical data**

Anordnung zur Echtzeit-Messung, Speicherung, Wiedergewinnung und Analyse von urodynamischen Parametern

Système de mesure on-line, de mémorisation, de restauration et d'analyse de données urodynamiques

(84) Designated Contracting States:
**AT BE CH DE DK ES FR GB GR IT LI NL SE**

(43) Date of publication of application:
**03.08.1994 Bulletin 1994/31**

(73) Proprietor: **MEDIDENTA B.V.**
**6245 LN Eijsden (Oost Maarland) (NL)**

(72) Inventor: **Rollema, Harm Jan**
**NL-6245 LN Oost-Maarland (Eijsden) (NL)**

(74) Representative: **van Westenbrugge, Andries et al**
**Nederlandsch Octrooibureau**
**P.O. Box 29720**
**2502 LS The Hague (NL)**

(56) References cited:
**US-A- 4 063 548**        **US-A- 5 167 237**

• **BIOMEDIZINISCHE TECHNIK vol. 30, no. 4, April 1985, BERLIN (DE) pages 85 - 90 F. BEUTELSPACHER ET AL. 'Ein portables System zur kontinuierlichen stessfreien Aufzeichnung der Harnblasenmotorik'**
• **MEDICAL AND BIOLOGICAL ENGINEERING vol. 13, no. 3, May 1975, STEVENAGE (GB) pages 472 - 474 D.B. SMITH ET AL. 'A New Instrument for Computing the Urethal Resistance in Urodynamic Studies'**

**Description**

The invention relates to a urological diagnostic system for patients having prostatism symptoms or other symptoms associated with voiding disorders, said system comprising

- measuring means for measuring the rate of the fluid flow out of the bladder during voiding of the bladder,
- measuring means for measuring intravesical pressure inside the bladder,
- processing means connected to said measuring means for processing the signals generated by said measuring means.

Hundreds of thousands of operations are performed annually on benigly enlarged prostates. A significant percentage of all patients do not improve after the operation while others develop new difficulties such as incontinence and impotence. There is also a mortality rate associated with the operation. To decrease the risks of the treatment and to improve the diagnosis and the treatment the medical world needs systems which on the basis of measurements on the human body are able to assist the urologist in making a correct accurate diagnosis.

A prior art system of the type described in the first paragraph, destined to provide data for supporting the urologist in making his diagnosis, is known from the article "A new instrument for computing the urethral resistance in urodynamic studies", by D.B. Smith et al published in Medical and Biological Engineering, Vol 13, no 3, may 1975, Stevenage (GB). As described in this article, the actual human body is simplified such that the urethra is considered as a rough pipe in which the fluid flow is completely turbulent. Under these circumstances it can be assumed that the friction factor f of the pipe is a measure of the resistance to flow and is proportional to $\Delta p/R^2$ wherein $\Delta p$ is the pressure inside the bladder measured by said second measuring means and R is the flow rate measured by said first measuring means. An analogue computer is used to process the two input signals R and $\Delta p$ into an output signal which represents $\Delta p/R^2$. If this signal is plotted graphically as function of time than for a non-obstructed case a rather smooth curve will be obtained whereas in an obstructed case the curve is more or less irregular.

Some experienced urologists claim to be able to analyse this type of data and differentiate between "seriously obstructed patients" and "certainly non-obstructed patients". Such assessment, however, is based on visual subjective inspection of the shape of the plot, without any quantitative specification. The treatment decision is thus based on this subjective impression of the treating urologist and eventually other clinical observations and tests including rectal palpation, residual urine assessed by ultrasound of the bladder, ultrasound of the prostate, free uroflowmetry and optional cystoscopy. None of these examinations, however, supply an objective, quantitative measure to determine either the degree of obstruction or the degree of bladder failure.

The abovementioned resistance factor $\Delta p/R^2$ is based on hydrodynamics principles of a "stiff rigid rough pipe" and such a simple model does not take into account viscoelastic properties of the urethra and the urinary bladder contractility. Therefore an accurate diagnosis on the basis of this resistance factor, which is not the actual "urethral resistance", is proven to be hardly possible.

The attention is drawn to the fact that the above mentioned resistance factor is not equal to the so-called "urethral resistance".

Traditionally the field of urology has not focused its attention on differential diagnosis of "outflow obstruction" and "bladder failure" in order to assess on the one hand candidates for prostatectomy (which as used herein includes transurethral prostatic resection or "turp") and on the other hand candidates for other treatments.

With the known prior art systems even experienced urologists cannot objectively assess the indication for therapeutic interventions or evaluate their effect since it does not supply validated parameters to quantify urethral resistance and detrusor contractility. This is also true for gynaecologists who at present have no objective means to make a differential diagnosis between women with voiding complaints due to "urinary outflow obstruction" and/or impaired detrusor contractility ("detrusor"= urinary bladder).

It is now an object of the present invention to provide a computer-controlled system

- for preoperative assessment of male prostatectomy or female bladder neck incision candidates,
- for objective and selective quantification of bladder contractility and urethral resistance in men and women,
- for accurate separation of patients with bladder outflow obstruction from those with detrusor contractility failure,
- for predicting the clinical results of contemplated surgical procedures (such as prostectomy, bladder neck incision and anti-incontinence surgery), non-surgical therapeutical procedures (such as pharmaceutical treatment, hyperthermia, balloon dilatation and prostatic stents, as well as for evaluating bladder contractility before and after any such procedure,

for objective quantitative assessment of the effectiveness of new therapeutical methods and techniques for treatment of micturition disorders, caused by outflow obstruction and/or detrusor contractility failure.

for direct on-line measurement and quantification of a patient's urethral resistance and detrusor contractility.
for on-line measurement, storage, retrieval and analysis of urodynamic data for preoperative and postoperative assessment of both male and female urological disorders, caused by outflow obstruction and/or detrusor contractility failure.

To fulfil at least part of said objects the invention now provides a system of the type mentioned in the first paragraph which according to the invention is characterised in that the system comprises further the features as defined in present claim 1.

Said parameters calculated from the various recorded signals according to algorithms, which are known as such, are for the medical attendant very reliable means to decide if a patient has an obstruction of the bladder and needs an operation or has no obstruction but impaired detrusor contractility and needs medical care in another fashion.

By measuring both the intra-abdominal pressure as well as the pressure inside the bladder the solaced detrusor pressure can be derived. The generally accepted definition reads: "detrusor pressure is bladder pressure minus intra-abdominal pressure". Calculating parameters based on the detrusor pressure instead of bladder pressure leads to far more accurate and reliable results.

The intra abdominal pressure measuring means can be embodied as means for measuring the pressure inside the rectum of the patient. However, various other means are known for measuring the intra-abdominal pressure. In general a rectal catheter is to be preferred because it causes very little inconvenience for the patient With respect to the algorithms used for calculating the above-mentioned parameters the attention is drawn to the following prior art publications:

- "Quantification of urethral resistance and bladder function during voiding, with special reference to the effects of prostate size reduction on urethral obstruction due to benign prostatic hyperplasia" by Griffiths D.J, van Mastrigt R. and Bosch R. in Neurol. Urodynam. 8: 17-27, 1989.
- "Urinary bladder function and its control in healthy females", by Griffiths D.J. Constantinou C.E., and van Mastrigt R.,in Am.. J. Physiol. 251: R225-R230, 1986.
- "Electric stimulation of smooth muscle strips from the urinary bladder of the pig" by van Mastrigt R. and Glerum J.J. in J. Biomed. Eng. 7:, 2-8,1985.

It will be clear that the fluid volume measuring means and the supply means allow access to the volume of fluid left in the bladder after voiding, the solace "residual urine". This is a further parameter used in the above-mentioned calculations.

The parameter mentioned under a1) of present claim 1 can be calculated in various ways. However, it is preferred that in a plot of the detrusor pressure as function of the flow rate a quadratic function closely fitting against the underside of said plot is used to obtain the parameter (URA) as cross-section of said quadratic function with the detrusor pressure axis.

Because of the valuable additional information it provides it is furthermore preferred that during calculation of the parameter mentioned under a2) of present claim 2 a plot of the contractility (W) of the bladder as function of the momentaneous bladder volume is determined.

To obtain further relevant information it is preferred that the processing means furthermore calculates or determines at least a number of the following parameters:

- Qmax being the maximum flow rate during the voiding period
- Wmax being the maximum value of W in a plot of the contractility (W) of the bladder as function of the momentaneous bladder volume is determined.
- W(Qmax) being the value of the detrusor contractility at the moment of the maximum flow rate Qmax,
- rV(Qmax) being the relative volume at which W(Qmax) occurred,
- W20 being the value of the detrusor contractility W at a relative volume rV=0,20,
- W80 being the value of the detrusor contractility W at a relative volume rV=0,80,
- Pdet(max) being the maximum detrusor pressure

## BRIEF DESCRIPTION OF THE DRAWINGS

For a more complete understanding of the present invention and the advantages thereof, reference should be made to the following Detailed Description taken in connection with the accompanying drawings in which:

FIGURE 1 is a simplified block diagram of a computerized system for assessing urological disorders according to the present invention;
FIGURE 2A is a flowchart representation of a flow rate analysis routine used in the processing system of FIGURE 1;
FIGURE 2B is a flowchart representation of a pressure analysis routine used in the processing system of FIGURE 1;

FIGURE 3 is a representative plot of pressure as a function of flow rate generated by the control program of the present invention; and

FIGURE 4 is a representative plot of detrusor contractility as a function of bladder volume during micturition generated by the control program of the invention.

## DETAILED DESCRIPTION

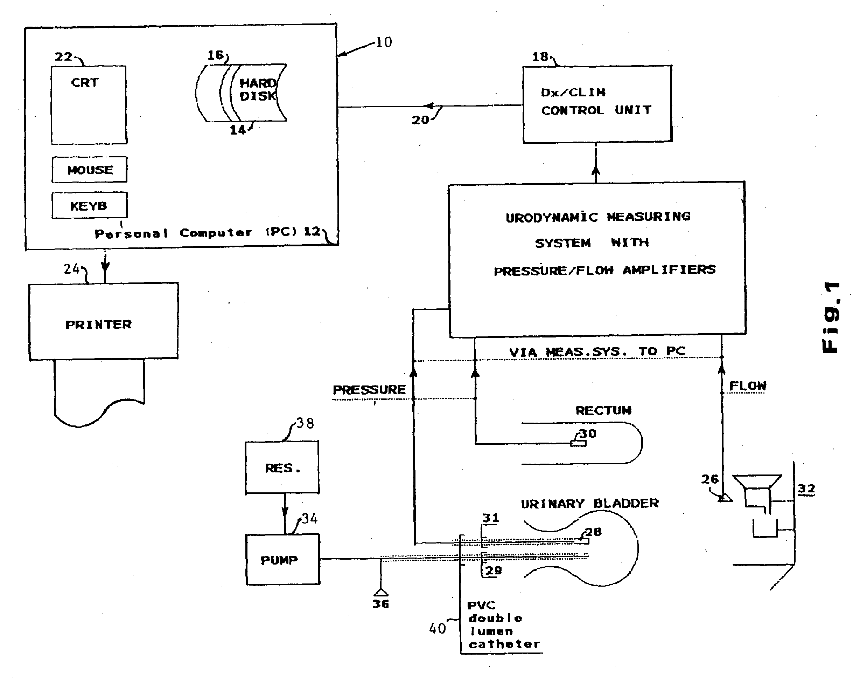

Referring now to FIGURE 1, a computer system 10 of the present invention comprises a digital processor such as an IBM-compatible personal computer 12 with appropriate hard disk storage 14 and associated disk drive 16. A control unit 18 for the system 10 is connected to the personal computer 12 preferably, but not necessarily, via an infrared or wireless transmission link 20. The personal computer 12 has a conventional video display terminal 22 and the system also preferably includes a 4-6 channel penwriter 24. The various input signals are provided to the system 10 via a flow transducer 26 and a pair of (microtip) pressure transducers 28 and 30 for measuring intravesical and intrarectal pressure. The system includes appropriate amplifying and other signal processing circuitry. To enhance response time, the flow transducer 26 preferably measures flow rate directly as opposed to deriving the value from volume measurements. One suitable product is the rotating disk transducer, Dantec Model No. Urodyn 1000. The flow rate transducer 26 is supported in a suitable micturition stand 32. A (peristaltic) pump 34 and filling volume sensor 36 are supported on the stand 32. A reservoir 38 filled with a test fluid, e.g., saline, is connected to the pump 34.

On-line measurement of urodynamic data is facilitated in the clinical environment through the use of one or more catheters. In particular, if a transurethral route is taken, then a pair of catheters is required. One catheter is used as a filling catheter to fill the bladder while the other catheter supports the pressure transducer 28. (Actually, the pressure transducer 28 is supported on the outer end of the catheter and the catheter is made air-tight such that an indirect pressure measurement is effected). The filling catheter, inserted via the transurethral route, is connected to the reservoir 38 through the pump 34. In particular, the patient to be tested is given a local anesthetic, and the catheter is inserted via the urethra into the bladder. The second (microtip) pressure measuring catheter is also inserted via the urethra into the bladder. Prior to micturation, the filling catheter is removed.

Alternatively, it is possible to use a suprapubic route as opposed to the transurethral route, in which case only one catheter is required. The bladder is filled via the suprapubical transcutaneous route by puncturing the bladder with the aid of a cystocath, $\pm 2.0$ c.m. above or below the pubic bone, after injection of a local anesthetic into the skin at the puncture location. As seen in FIGURE 1, the catheter is preferably a 9 Fr-PVC suprapubic double lumen catheter having a first channel 29 and a second channel 31. The first channel 29 is connected to the saline pump 34 and the second channel 31 is used to support one of the microtip pressure transducers 28. As will be described, the invention uses for analysis the subtracted detrusor pressure (intravesical pressure minus intrarectal pressure). The other pressure transducer 30 is supported in the patient's rectum and used as a measure of intraabdominal pressure, which acts as a reference to the intravesical pressure measured by the pressure transducer 28.

The preferred testing protocol is as follows. Three times in succession the patient's bladder is filled with saline, at medium filling rate (e.g., 40 ml/min), via the first channel of the catheter. One test may be sufficient as well (i.e., one filling/voiding cycle). When the patient experiences an urge to void, he/she voids into the flow transducer 26. Via a switch box that is part of the control unit 18, the control program (as will be described) is triggered to begin and then cease storage of relevant signals. These signals include pressure and flow rate during micturation or bladder contraction, as well as the isometric detrusor pressure just before flow started. If the signals are in analog form, they are converted to digital form (by a suitable analog-to-digital converter) and stored in the database 14. The penwriter 24 outputs some or all of the monitored signals including intravesical pressure (from transducer 28), intraabdominal pressure (from transducer 30), subtracted detrusor pressure (the output of transducer 28 minus the output of transducer 30), flow rate (from transducer 26), etc. By recording such signals on the penwriter 24, the video display 22 is left free for displaying other more pertinent data as well be described. After voiding, residual urine is also measured directly by emptying the bladder via the double lumen catheter or via the transurethral filling catheter.

The computer processing system includes a software control program supported on the personal computer 12, for retrieving, displaying and analyzing such urodynamic data to assist the clinician in characterizing the patient's true physical condition. In part, the software control program facilitates accurate separation of patients with bladder outflow obstruction from those with detrusor contractility failure. Differential diagnosis of such conditions enable the clinician to more accurately predict the clinical result of contemplated surgical procedures, such as prostatectomy, and of non-surgical procedures. The program also enables objective quantitative assessment of the effectiveness of new therapeutical methods and techniques for treatment of micturition disorders.

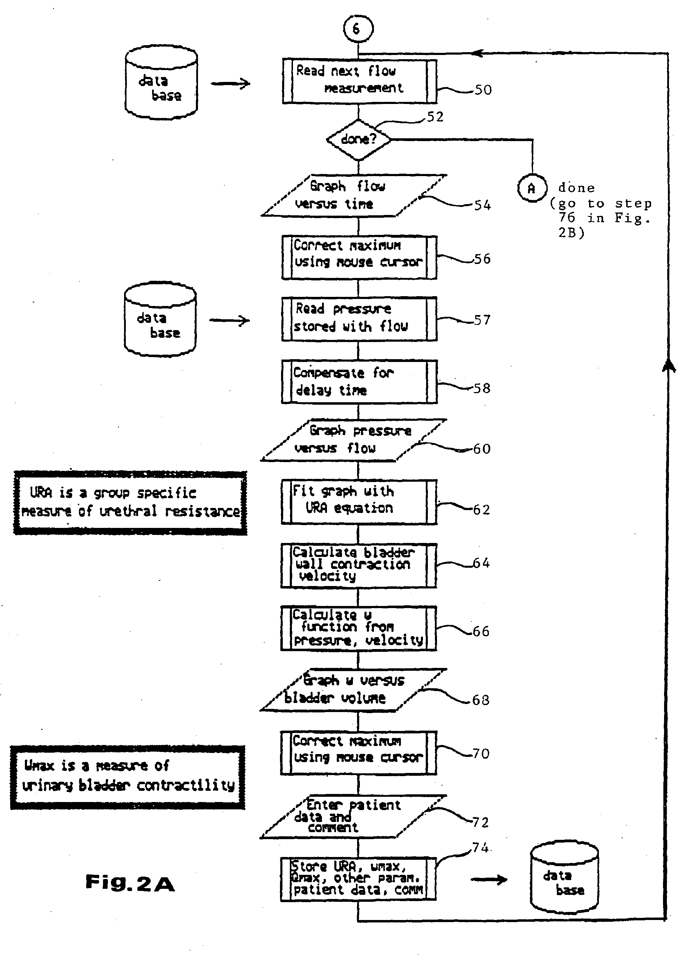

Referring now to FIGURE 2A, the analysis routine begins after an appropriate number (e.g., three (3) but minimally one) of bladder filling/voiding tests are run and the resultant urodynamic signals are digitized and stored in the database. At step 50, the routine retrieves from the database 14 a flow rate measurement from one of the preferably three (3) tests. A test is done at step 52 to determine if the flow rate measurement has been previously evaluated. If not, the routine then continues at step 54 to generate a plot of flow rate versus time. This plot is displayed on the video terminal 22 at step 54. The routine then identifies and displays on the screen a flow rate value considered to be a maximum, and identified as a variable $Q_{max}$. The clinician can then correct the proposed value using a cursor of the personal computer 12. This correction is effected at step 56 and serves to eliminate artifacts, which may occur due to measured errors, e.g., interference with the flow transducer 26. The routine then continues at step 57 to retrieve the pressure data corresponding to the flow rate measurement being analyzed. At step 58, the routine compensates for the time delay that occurs between the pressure and flow rate measurements. Such delay occurs because the flow transducer is separated from the pressure transducer by approximately 0.5 meter in the urodynamic setup and the flow rate measured at a certain moment is related to the detrusor pressure measured earlier in time. To compensate for this lag time, the detrusor pressure is right-shifted a predetermined amount, i.e., equal to approximately 0.5 - 0.8 seconds, on the display.

## OBSTRUCTION PARAMETER URA

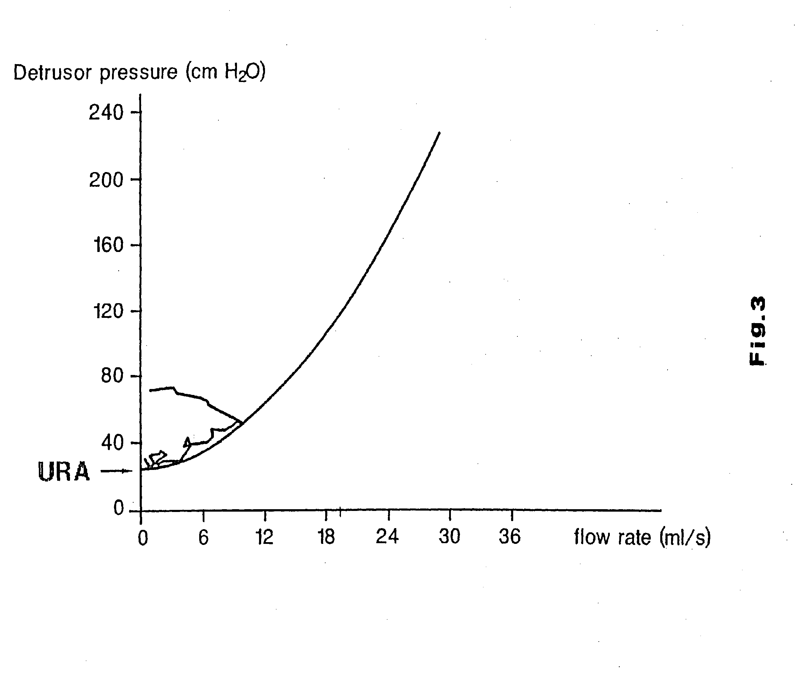

At step 60, a plot of pressure versus flow rate is generated and displayed on the video display 22. One such plot is shown, for example, in FIGURE 3. The routine continues at step 62 by fitting the pressure/flow rate plot generated in step 60 with a predetermined quadratic urethral resistance relation set forth below. The quadratic resistance relation is characterized by two (2) parameters: its intercept on the pressure (i.e, the "y") axis, and its curvature. Experimentally, it has been established that a group-specific relation exists between these two parameters, i.e., that steeper curves generally also intercept the pressure axis at a higher value. This means that within a group of patients (e.g., adults), the urethal resistance can be characterized with one parameter only. This one parameter can be calculated from one representative point in the pressure flow data, e.g., the point of maximum flow. Arbitrarily, the intercept on the pressure axis was chosen as the one representative parameter.

In particular, at step 62 the routine determines the intersection of the quadratic resistance relation with the pressure axis of the pressure/flow rate plot. This intersection, arbitrarily selected as the representative parameter (as described above), is then defined as the urethral resistance parameter "URA." URA is a group specific measure (valid for a specific group of patients, males, females, etc.) of urethral resistance.

## Contraction Strength Variable W

Of the various methods of assessing the strength of a detrusor contraction during voiding, the most attractive is the calculation of the external mechanical power generated (detrusor pressure x flow rate). However, this value is dependent on the volume in the bladder and is not constant for a constant contraction strength. It falls to zero if the contraction is isovolumetric (i.e., if detrusor pressure - 0). Therefore a modified form has been adopted in which these objections are overcome.

Specifically, the contraction of the detrusor is governed by the so-called Hill equation, which describes the relation between the tension developed and the velocity of shortening of the contracting muscle. The equation may be written in terms of the pressure $P_{det}$ developed by the complete bladder (which is assumed to be spherical) and the velocity of shortening of the detrusor circumference $v_{det}$ as:

$$(P_{det} + a) \ (V_{det} + b) = 5 \ ab \qquad (A1)$$

Where "a" and "b" are experimentally-derived contractility constants. The approximate median values for a and b are 25 cm ($H_2O$) and 6 mm/s, respectively. The above form of the equation is convenient because its two adjustable parameters, the isovolumetric detrusor pressure and the physiological maximum value of the detrusor shortening velocity, are normally roughly independent of the volume in the bladder. Thus the left-hand side of equation Al is approximately constant for fixed contraction strength, irrespective of the volume in the bladder, and it also increases with increase of $P_{det}$ and/or $v_{det}$. It is therefore a possible volume-independent measure of contraction strength. To ensure a value of zero when there is no contraction at all, it is appropriate to subtract ab from the left-hand side of equation Al. Because $P_{det}$ x $v_{det}$ is approximately equal to 2 times the external mechanical power developed by the detrusor divided by the surface area of the bladder, it is appropriate to divide the resulting function by $2\pi$. The resulting measure of detrusor contraction strength is thus:

$$WF = [(P_{det} + a) \ (v_{det} + b) - ab]/2\pi \qquad (A2)$$

WF may be considered approximately as the mechanical power per unit area of bladder surface developed by the contracting detrusor, modified to allow for the finite power necessary to sustain an isovolumetric contraction or to shorten at high velocity under zero load.

The detrusor shortening velocity variable $v_{det}$ is calculated from the equation:

$$v_{det} = Q/2[3(V + V_t)/4\pi]^{2/3} \quad (A3)$$

in which the bladder is treated as a thick-walled sphere having a lumen of volume V. Q is the measured volume flow rate of urine, and $V_t$ represents the volume of noncontracting tissue enclosed by the effectively contracting detrusor tissue near the end of bladder emptying. If the bladder is not spherical, then $v_{det}$ represents an average velocity of shortening of the detrusor circumference. V is calculated by integrating Q backward from the end of voiding, allowing for any residual urine. In principle, the value of $P_{det}$ used in equation AI should be delayed by 0.5-0.8s in order to allow for the time delay in measuring Q.

To calculate WF, approximate median values for a and b (25 cm $H_2O$ and 6 mm/s), respectively, are used. Experimental tests showed that the value of WF was not very sensitive to changes in these assumed values. Estimates of $V_t$ have ranged from 2 to 50 ml. Changes in this value affect WF significantly one at the end of voidings with little residual urine. If too small a value of $V_t$ is assumed, WF may fluctuate significantly under these circumstances. The anatomically reasonable value of 10 ml eliminates such artifacts and is used in the calculations.

WF can be used to measure detrusor contraction strength during both voiding and filling. During filling WF is directly proportional to $P_{det}$ (see equation A2). With the assumed value of b, WF (in W/m$^2$ or $\mu$W/mm$^2$) is approximately equal to 0.1 $P_{det}$ (in cm $H_2O$).

**Further Considerations Concerning the Above Models**

The more-detailed models describing muscle contraction on the basis of cross-bridge interaction between actin and myosin filaments developed below can be shown to yield a force-velocity relation which is mathematically differently formulated, but approximates a hyperbola very closely. Such a hyperbolic force-velocity relation is characterized by three parameters: $F_0$, the intercept with the force axis, or the maximum isometric force the muscle can bare; $v_{max}$, the intercept with the velocity axis, or the maximum (unloaded) contraction velocity; and $aF_o^{-1}$, the degree of curvature of the hyperbola.

For the urinary bladder muscle and many other types of muscle $aF_o^{-1}$ is generally found to be a constant of approximately 0.25 so that two parameters, $F_o$ and $v_{max}$, completely characterize the relation between force and shortening velocity of this muscle. Both variables depend severely on the length of the muscle. $F_o$ depends on muscle length, showing a clear optimum "working length" of the muscle, and $v_{max}$ shows a similar length dependence. In striated muscle these length dependencies are explained by varying degrees of overlap of the actin and myosin filaments, which directly influence the number of cross-bridges that can be formed.

For smooth muscle a similar mechanism has been proposed, but since a regular filament arrangement is lacking in this type of muscle, there is no basis for such a mechanism. Indeed a length-dependent activation of smooth muscle has been proposed to account for the observed length dependencies. Apart from their dependence on length, $F_o$ and $v_{max}$ also depend on the degree of activation of the muscle. In an inactive muscle, both $F_o$ and $v_{max}$ are zero, and during the onset of stimulation both parameters somehow increase to a maximum that is maintained for some time depending on stimulus conditions. It is this maximum that is representative for the (myogenic) contractile properties of the muscle.

Power itself is not a useful variable to represent contraction strength as it is zero for zero pressure or zero flow rate. As a consequence, the power in a very high isometric contraction is zero, as is the power in a voiding with high flow rate at (almost) zero pressure, which is often seen in normal females. A proposed variable, W, overcomes this problem as it contains additive terms based on Hill's equation that make it non-zero if either flow rate or detrusor pressure is non-zero. In the normal case W slowly rises on bladder emptying as a result of the length dependence of $F_o$ and $v_{max}$. In case of failing voiding, W decreases prematurely, leaving residual urine.

The maximum of W during bladder emptying can be taken as a parameter of contractility; $W_{max}$ equals the product of $F_o$ with $V_{max}$ and a constant.

The method of the present invention takes advantage of the above-identified models. Referring now back to FIGURE 2A, at step 64, the routine calculates bladder wall contraction velocity and at step 66, the routine calculates a detrusor contractility strength function from the bladder wall contraction velocity calculated in step 64 and from the pressure data retrieved at step 57.

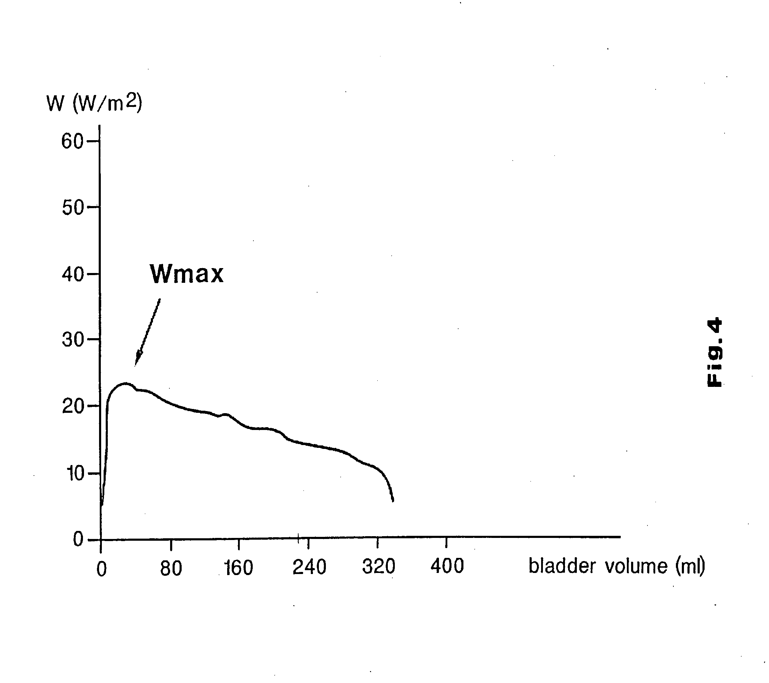

The routine then continues to step 68 to plot the contractility strength function versus bladder volume, as shown in representative FIGURE 4. This plot represents detrusor contractility throughout micturition. The routine again indicates a proposed maximum value $W_{max}$ which the user can then correct (by inspecting the plot) at step 70 using the cursor. The patient data is then entered at step 72, and this data and the following urodynamic parameters are stored at step 74:

- Qmax: maximum flowrate
- Wmax: detrusor contractility parameter
- URA: obstruction parameter
- rV (Wmax): the relative volume at which Wmax occurred
- W (Qmax): the value of W at the moment of maximum flowrate
- W20: the value of W at a relative volume rV=0.20
- W80: the value of W at a relative volume of rV=0.80
- $P_{det}$ (max): the maximum detrusor pressure
- Residual urine: the amount of urine that is left in the bladder after micturition

The routine then returns to step 50 to retrieve the next detrusor pressure and flow rate measurement, i.e. from the next test.

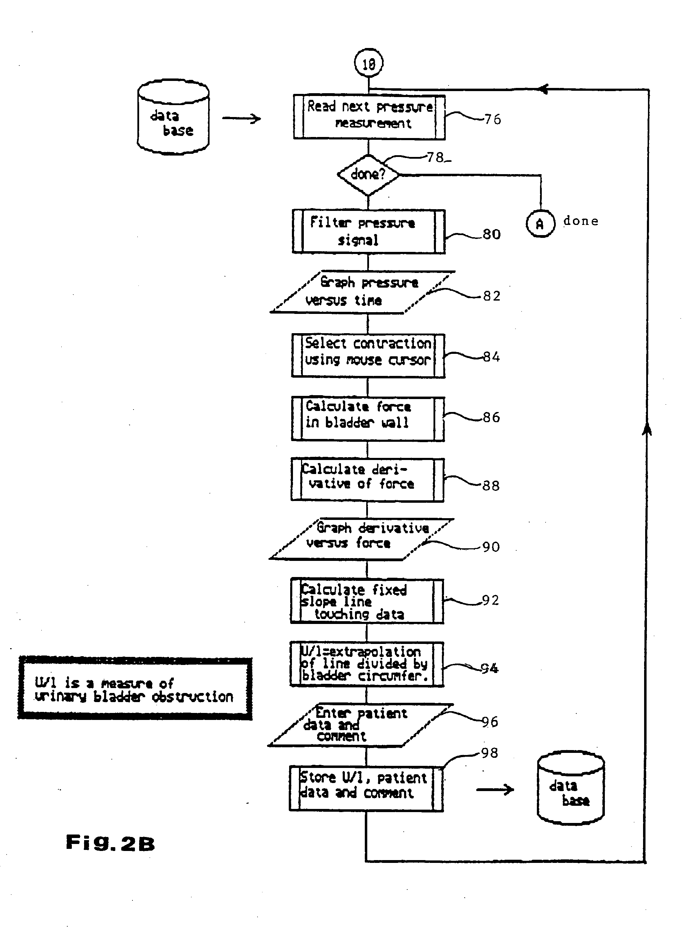

After the flow rate measurements for the three tests have been processed in this manner, the user may continue as shown in FIGURE 2B. In particular, at step 76, the routine reads the oldest unprocessed contraction measurement from the database. The routine filters the signal at step 80 with a moving low pass digital filter. The purpose of this filtering is to remove artifacts, e.g. electrical spikes, without losing essential information. A plot of pressure versus time is then generated and displayed at step 82.

**Obstruction Parameter U/l and Bladder Force**

At step 84, the beginning and the end of the contraction are selected using the cursor. In particular, the screen shows the preceding part of the pressure signal before the contraction starts, the rise of the contraction and stops at the moment the flow rate starts. By moving the mouse cursor the operator indicates the beginning of the rising part and the end of the contraction, in fact the end of the signal (the point where flow rate starts).

Using this selected isometric portion of the contraction, the routine continues at step 86 and calculates the bladder wall force as a function. The derivative of the bladder wall force is then calculated at step 88. The routine then plots the derivative versus force at step 90.

If the series elasticity of smooth muscle formed a constant, i.e. obeyed one unchanging relation between its length and force, it would be possible to calculate properties of the contractile element from the time course of the development of isometric force of pressure in the urinary bladder. Unfortunately, as described above this is not the case. Alternatively, it has been shown by phase plot analysis that isometric force development in vitro is related to activation of the contractile element rather than its shortening. In phase plots made from clinically measured isometric contractions, i.e. the detrusor pressure rise before the onset of micturition, a straight line part is much more difficult to recognize than in phase plots from isometric contractions measured in vitro. By fitting a straight line with preset slope to such "clinical" phase plots, a parameter (U) was obtained that was normalized by dividing it by the bladder circumference. The normalized parameter U/1 was extensively evaluated.

From this evaluation, it was concluded that the parameter U/l derived from phase plots of the isometric detrusor pressure rise before the outset of micturition is biased by the urethral opening pressure to such a degree that it can be used as a measure for the degree of obstruction. Here it has been concluded that under clinical circumstances the information in the isometric development of pressure in the urinary bladder before the outset of micturition is very useful, but not necessarily with respect to measuring contractility.

Referring back to FIGURE 2B, at step 92, the routine calculates a fixed slope line touching the derivative versus force curve. The parameter "U/l" is then calculated at step 94 by extrapolating the fixed slope line divided by a bladder circumference value. Experimental observation has shown this parameter to be linearly dependent on 1 (as described above). Therefore the parameter U is normalized by dividing it by 1. The U/l parameter is a measure of urinary bladder outflow obstruction. Patient data is then entered at step 96, and the U/l parameter and such patient data are stored at step 98.

The routine then recycles to obtain the next pressure measurement.

The URA, U/l and $W_{max}$ parameters have been found to possess significant clinical predictive capability. A value of URA larger than a predetermined amount indicates "obstruction" ("OBS"). A value less than this amount indicates a non-obstructed ("NOBS") condition. Thus, based on the OBS or NOBS conclusion, the clinician can differentially diagnose or separate patients with bladder outflow obstruction from those with detrusor contractility failure. Based on clinical evaluations, the urethral resistance parameter URA has a sensitivity of approximately 90% and a specificity of 97% for this purpose. The U/l parameter, while technically a measure of bladder contractility, also possesses a significant predictive capability for diagnosing an OBS condition because it effectively reflects the isometric detrusor pressure needed to overcome an obstruction, and more initial pressure will be required if such a condition exists. A value of U/l

> 54 W/m$^2$ indicates the presence of obstruction, while a value less than this amount makes the existence of an obstruction unlikely.

If both parameters (URA and U/l) are considered a straight line according to the equation:

$$U/1 + 2.58 \cdot URA \leqslant 128,$$

then such time as plotted separates the OBS from the NOBS patients. If both obstruction parameters URA and U/I are considered clinical data have shown that the confirmation has a sensitivity and specificity both of almost 100%.

A value of $W_{max}$ less than a predetermined amount (12.85 W/m$^2$) indicates detrusor contractility lower than normal. The $W_{max}$ parameter also accurately predicts the likelihood of residual postoperative urine in patients with low detrusor contractility; specifically, if $W_{max}$ is preoperatively less than the above-mentioned cutoff value, there is a high likelihood of residual urine post-operatively. The parameter $W_{max}$ characterizes urinary bladder contractility.

It should be appreciated by those skilled in the art that the specific embodiments disclosed above may be readily utilized as a basis for modifying or designing other structures for carrying out the same purposes of the present invention. For example, it is envisioned that other measures of urethral resistance and detrusor contractility be used in conjunction with the computerized system of the present invention. It should also be realized by those skilled in the art that such equivalent constructions do not depart from the scope of the invention as set forth in the appended claims.

**Claims**

1.   Urological diagnostic system for patients having prostatism symptoms or other symptoms associated with voiding disorders, said system comprising

- measuring means (26, 32) for measuring the rate of the fluid flow out of the bladder during voiding of the bladder,
- measuring means (28) for measuring intravesical pressure inside the bladder
- processing means (10, 18, 20) connected to said measuring means (26, 28) for processing the signals generated by said means

**characterized in that** the system comprises further

- measuring means (30) for measuring the intra-abdominal pressure,
- measuring means (36) for measuring the volume of fluid flowing out of the bladder during voiding of the bladder,
- fluid supply means (29, 34, 36, 38) for supplying a predetermined volume of fluid into the bladder,
- said intra-abdominal pressure measuring means (30) and said volume measuring means (36) also being connected to said processing means,
- said processing means being embodied such that, after a predetermined volume of fluid is supplied into the ladder and during the succeeding voiding period the signals of all precited measuring means are recorded, said processing means will calculate based on known formulas at least one of the following parameters:

- a1) a parameter (URA) being indicative for the urethral resistance
- a2) a parameter (Wmax or U/l) being indicative for the detrusor contractility of the bladder.

2.   System according to claim 1, **characterized in that** during the calculation of the urethral resistance parameter (URA) in a plot of the detrusor pressure (difference between pressure ion the bladder measured by the second measuring means (28) and the intra-abdominal pressure measured by the third measuring means (30)) as function of the flow rate, a quadratic resistance relation closely fitting against the underside of said plot is used to obtain the parameter (URA) as intersection of said quadratic relation with the detrusor pressure axis.

3.   System according to claim 1, **characterized in that** during calculation of the detrusor contractility parameter (Wmax or U/l) a plot of the detrusor contractility of the bladder as function of the momentaneous bladder volume is determined.

4.   System according to one of the preceding claims, **characterized in that** the processing means furthermore calculates or determines at least a number of the following parameters:

- Qmax being the maximum flow rate during the voiding period

- Wmax being the maximum value in a plot of the detrusor contractility (W) of the bladder as a function of the momentaneous bladder volume
- W(Qmax) being the value of the detrusor contractility at the moment of the maximum flow rate Qmax,
- rV(Wmax) being the relative volume at which Wmax occurred,
- W20 being the value of the detrusor contractility W at a relative volume rV = 0.20,
- W80 being the value of the detrusor contractility W at a relative volume rV = 0.80,
- Pdet(max) being the maximum detrusor pressure.

**5.** System according to one of the preceding claims, **characterized in that** said fluid supply means comprise a reservoir (38) containing the fluid, a catheter (29) one end being connected to said reservoir (38) and the other end extending into the bladder, and a pump (34) for creating a flow of fluid from the reservoir (38) through said catheter (29) into the bladder.

**Patentansprüche**

**1.** Urologisches Diagnosesystem für Patienten mit Prostatismussymptomen oder anderen Symptomen in Zusammenhang mit Entleerungsstörungen, wobei das System folgendes umfasst:

- Messmittel (26, 32) zum Messen der Geschwindigkeit der Flüssigkeitsströmung aus der Blase heraus während des Entleerens der Blase,
- Messmittel (28) zum Messen des intravesikalen Drucks innerhalb der Blase,
- Verarbeitungsmittel (10, 18, 20), die mit den Messmitteln (26, 28) verbunden sind, um die durch die Messmittel erzeugten Signale zu verarbeiten,

**dadurch gekennzeichnet,**
**dass** das System ferner folgendes umfasst:

- Messmittel (30) zum Messen des intraabdominalen Drucks,
- Messmittel (36) zum Messen des Volumens von Flüssigkeit, die während des Entleerens der Blase aus der Blase fließt,
- Flüssigkeitszufuhrmittel (29, 34, 36, 38) zum Zuführen eines vorbestimmten Volumens von Flüssigkeit in die Blase,
- wobei die Intraabdominaldruck-Messmittel (30) und die Volumenmessmittel (36) auch mit den Verarbeitungsmitteln verbunden sind,
- wobei die Verarbeitungsmittel derart ausgestaltet sind, dass, nachdem ein vorbestimmtes Volumen von Flüssigkeit in die Blase zugeführt worden ist und während des darauffolgenden Entleerungszeitraums, die Signale aller zuvor genannten Messmittel aufgezeichnet werden, wobei die Verarbeitungsmittel auf der Grundlage von bekannten Formeln mindestens einen der folgenden Parameter berechnen:
- a1) einen Parameter (URA), der den urethralen Widerstand anzeigt,
- a2) einen Parameter (Wmax oder U/l), der die Detrusor-Kontraktionsfähigkeit der Blase anzeigt.

**2.** System nach Anspruch 1, **dadurch gekennzeichnet, dass** während der Berechnung des Parameters für den urethralen Widerstand (URA) in einer grafischen Darstellung des Detrusor-Drucks (Unterschied zwischen dem Druck in der Blase, gemessen durch das zweite Messmittel (28), und dem intraabdominalen Druck, der durch das dritte Messmittel (30) gemessen wird) als Funktion der Durchflußgeschwindigkeit ein quadratisches Widerstandsverhältnis, das knapp an der Unterseite der grafischen Darstellung aufgetragen ist, verwendet wird, um den Parameter (URA) als Schnittpunkt der quadratischen Beziehung mit der Detrusordruck-Achse zu erhalten.

**3.** System nach Anspruch 1, **dadurch gekennzeichnet, dass** während der Berechnung des Parameters für die Detrusor-Kontraktionsfähigkeit (Wmax oder U/l) eine grafische Darstellung der Detrusor-Kontraktionsfähigkeit der Blase als Funktion des momentanen Blasenvolumens bestimmt ist.

**4.** System nach einem der vorhergehenden Ansprüche, **dadurch gekennzeichnet, dass** das Verarbeitungsmittel ferner mindestens eine Anzahl folgender Parameter berechnet oder bestimmt:

- Qmax als maximale Durchflußgeschwindigkeit während des Entleerungszeitraums,
- Wmax als maximalen Wert in einer grafischen Darstellung der Detrusor-Kontraktionsfähigkeit (W) der Blase als Funktion des momentanen Blasenvolumens,

- W(Qmax) als Wert der Detrusor-Kontraktionsfähigkeit zum Zeitpunkt der maximalen Durchflußgeschwindigkeit Qmax,

- rV (Wmax) als relatives Volumen, bei dem Wmax aufgetreten ist,

- W20 als Wert der Detrusor-Kontraktionsfähigkeit W bei einem relativen Volumen rV = 0,20,

- W80 als Wert der Detrusor-Kontraktionsfähigkeit W bei einem relativen Volumen rV = 0,80,

- Pdet(max) als maximaler Detrusor-Druck.

**5.** System nach einem der vorhergehenden Ansprüche, **dadurch gekennzeichnet, dass** die Flüssigkeitszufuhrmittel einen Behälter (38), der die Flüssigkeit enthält, einen Katheter (29), von dem ein Ende mit dem Behälter (38) verbunden ist und das andere Ende sich in die Blase erstreckt, und eine Pumpe (34) umfassen, um einen Flüssigkeitsfluss von dem Behälter (38) durch den Katheter (29) in die Blase zu erzeugen.

## Revendications

**1.** Système de diagnostic urologique pour des patients ayant des symptômes de prostatisme ou d'autres symptômes associés à des désordres de miction, ledit système comprenant :

un moyen de mesure (26,32), pour mesurer le débit d'écoulement de fluide de la vessie pendant la miction de la vessie ;

un moyen de mesure (28) pour mesurer la pression intravésicale à l'intérieur de la vessie

un moyen de traitement (10, 18, 20) raccordé auxdits moyens de mesure (26, 28) pour traiter les signaux générés par lesdits moyens,

**caractérisé en ce que** le système comprend en outre :

un moyen de mesure (30) pour mesurer la pression intra-abdominale,

un moyen de mesure (36) pour mesurer le volume d'écoulement de fluide de la vessie pendant la miction de la vessie ;

un moyen de fourniture de fluide (29, 34, 36, 38) pour fournir un volume de fluide prédéterminé dans la vessie, ledit moyen de mesure de pression intra-abdominale (30) et ledit moyen de mesure de volume (36) étant aussi raccordés audit moyen de traitement,

ledit moyen de traitement étant incorporé pour que, après qu'un volume prédéterminé de fluide soit fourni dans la vessie et que pendant la période de miction suivante les signaux de tous les moyens de mesure précités sont enregistrés, ledit moyen de traitement calcule sur la base des formules connues au moins un des paramètres suivants :

a1) un paramètre (URA) étant indicateur de la résistance urétrale

a2) un paramètre (Wmax ou U/l) étant indicateur de la contractilité du détrusor de la vessie.

**2.** Système selon la revendication 1, **caractérisé en ce que** pendant le calcul du paramètre de résistance urétrale (URA) dans un relevé de la pression du détrusor (différence entre la pression sur la vessie mesurée par le second moyen de mesure (28) et la pression intra-abdominale mesurée par le troisième moyen de mesure (30)) en fonction du débit, une relation de résistance quadratique s'adaptant étroitement contre le côté inférieur dudit relevé est utilisée pour obtenir le paramètre (URA) en tant qu'intersection de ladite relation quadratique avec l'axe de pression du détrusor.

**3.** Système selon la revendication 1, **caractérisé en ce que** pendant le calcul du paramètre de contractilité du détrusor (Wmax ou U/l) un relevé de la contractilité du détrusor de la vessie en fonction du volume de vessie momentané est déterminé.

**4.** Système selon l'une des revendications précédentes, **caractérisé en ce que** le moyen de traitement calcule ou détermine en outre au moins un nombre de paramètres suivants :

Qmax étant le débit maximum pendant la période de miction

Wmax étant la valeur maximum dans un relevé de la contractilité du détrusor (W) de la vessie en fonction du volume momentané de vessie

W(Qmax) étant la valeur de la contractilité du détrusor au moment du débit maximum Qmax,

rV(Wmax) étant le volume relatif auquel Wmax s'est produit,
W20 étant la valeur de la contractilité du détrusor W à un volume relatif rV=0,20,
W80 étant le volume de la contractilité du détrusor W à un volume relatif rV=0,80,
Pdet(max) étant la pression de détrusor maximum.

5. Système selon l'une quelconque des revendications précédentes,
   **caractérisé en ce que** le moyen de fourniture de fluide comprend un réservoir (38) contenant le fluide, un cathéter (29) une extrémité étant raccordée audit réservoir (38) et l'autre extrémité étant dans la vessie, et une pompe (34) pour créer un flux de fluide à partir du réservoir (38) à travers ledit cathéter (29) dans la vessie.

Fig.1

Fig.2A

(10)

data
base → Read next pressure
measurement —76

78— done? ──────→ (A) done

Filter pressure
signal —80

Graph pressure
versus time —82

Select contraction
using mouse cursor —84

Calculate force
in bladder wall —86

Calculate deri-
vative of force —88

Graph derivative
versus force —90

Calculate fixed
slope line
touching data —92

U/l=extrapolation
of line divided by
bladder circumfer. —94

**U/l is a measure of
urinary bladder obstruction**

Enter patient
data and
comment —96

Store U/l, patient
data and comment —98 → data
base

## Fig. 2B

Fig.3

EP 0 608 593 B1

Fig.4